# EUROPEAN PATENT APPLICATION

(11) **EP 1 024 195 A1**
(43) Date of publication of application: **02.08.2000**
(21) Application number: 99200261.8
(22) Date of filing: 28.01.1999
(51) Int. Cl.: C12N 15/82, C07K 14/08, A01H 5/00

(54) **Coat protein genes of potyvirus of capsicum spp. and plants transformed therewith**

(71) Applicant: Bejo Zaden B.V., 1749 ZH Warmenhuizen (NL)
(72) Inventor: Jomon, Joseph, p/a Department of Biochemistry, Bangalore 560 012 (IN); Savithri, H.S. p/a Department of Biochemistry, Bangalore 560 012 (IN)
(74) Representative: 't Jong, Bastiaan Jacobus

(57) **Abstract**

The present invention relates to a recombinant DNA molecule comprising one or more DNA sequences showing at least 80% homology to the coat protein gene and/or the mutant nuclear inclusion protein b (NIb) protein gene, optionally in combination with the 3'-untranslated region of a potyvirus, for example Pepper Vein Banding Virus (PVBV), functionally linked with regulatory sequences needed for gene expression in plant cells.

The invention further relates to a transformed plant cell comprising said recombinant DNA molecule, as well as to a transgenic plant comprising said transformed plant cells and seeds derived therefrom.

## Description

### FIELD OF INVENTION

The present invention lies on the field of the molecular characterization of Pepper Vein Banding Virus (PVBV), a member of the plant potyvirus group which is common in India and most likely in many other countries in the world, and the determination of the nucleotide sequence of a single partial Open Reading Frame (ORF), containing sequences of NIa-protease, NIb protein, Coat Protein (CP) and the 3'-Untranslated Region (UTR). The invention more specifically relates to the use of the above information for obtaining plants resistant to the virus.

### BACKGROUND OF THE INVENTION

Plant viruses are a serious problem for many agricultural crops. Chilli pepper (hot pepper), Capsicum frutescens, and bell pepper (or sweet pepper), Capsicum annuum, are two fruitvegetable crops that are grown in tropical and sub-tropical agricultural areas and under greenhouse conditions in moderate climates. The crops can be heavily infected by several viruses, belonging to the group of the geminiviruses (Geminiviridae), which are DNA-viruses, and the group of the potyviruses (Potyviridae), which are RNA-viruses. In several tropical countries more than ten different viruses belonging to these two groups can infect Capsicum species.

Potyviruses are transmitted by aphids which are also an important component of the "Murda syndrome" in India, which leads to more than 50 % yield loss in chilli pepper. One of the methods to control this devastating disease would be to develop transgenic chilli pepper and bell pepper varieties that are resistant to potyviruses. However, until now, none of the isolates, especially from India, has been characterized on a molecular level.

Pepper Vein Banding Virus (PVBV) has been reported to be the most prevalent potyvirus infecting chilli pepper in south India. Initial characterization of PVBV with respect to particle morphology, immunoassays and N-terminal amino acid sequence of the coat protein suggested it to be a distinct member of the potyvirus group. However, the complete amino acid sequence of the coat protein (CP) and 3'-untranslated region provide a better basis for the classification of potyviruses. In addition, molecular characterization is an important aspect in designing strategies for developing transgenic plants resistant to viruses and it helps to understand the mechanisms underlying the processes of virus infection.

It is therefore the object of the invention to obtain sequence information for the coat protein and 3'-untranslated region and use this information in the development of virus resistant plants.

### DISCLOSURE OF THE INVENTION

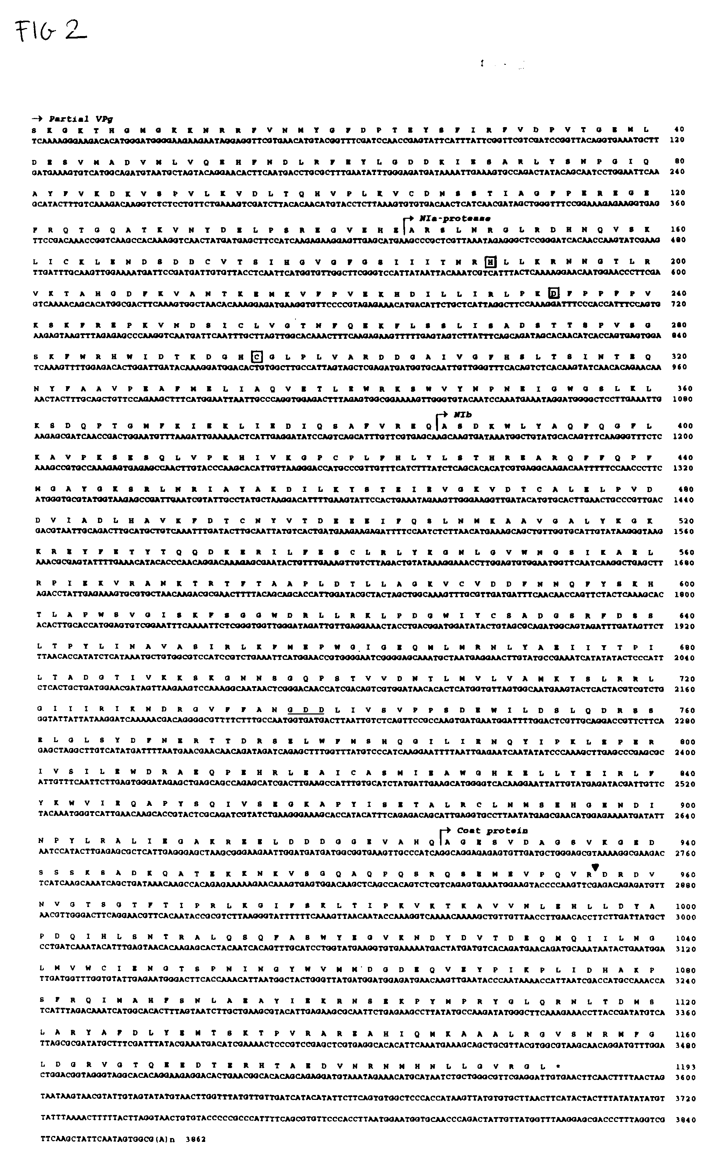

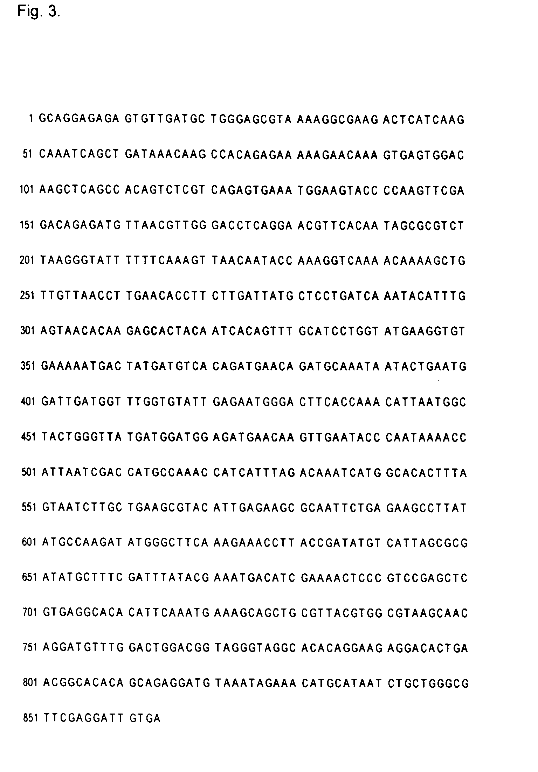

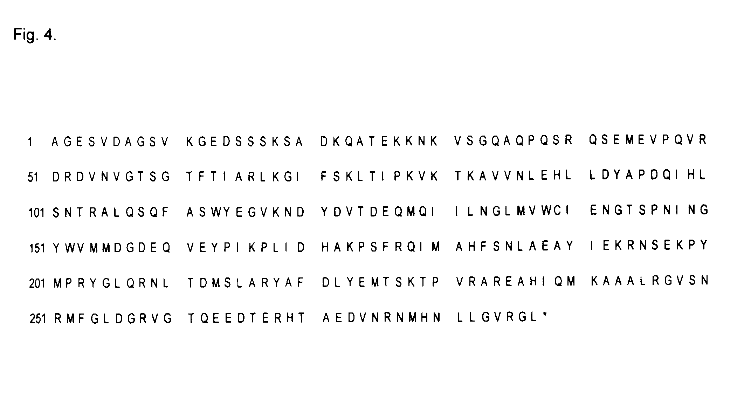

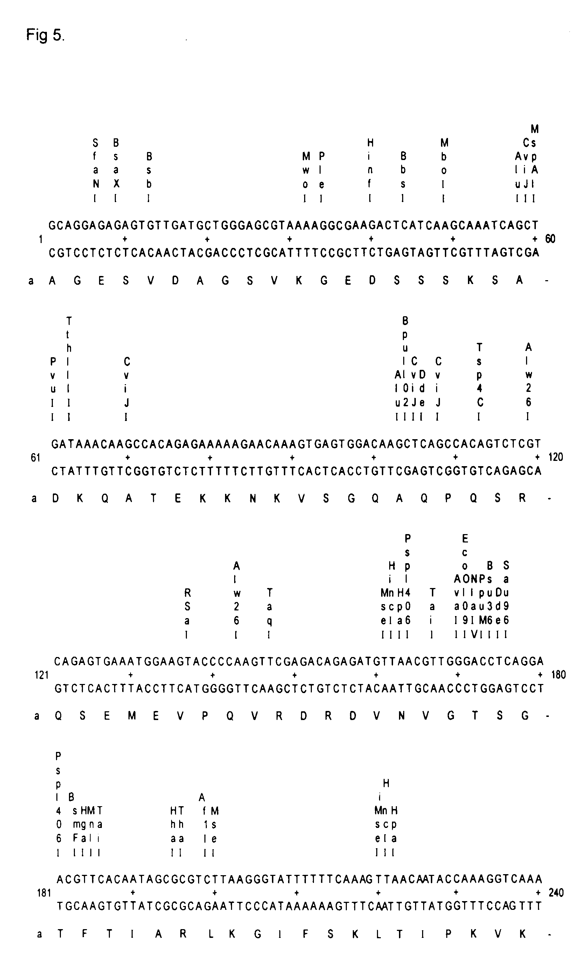

The research that led to the present invention has resulted in the isolation, purification and characterization of potyviruses, causing leaf and fruit damage in Capsicum frutescens and Capsicum annuum; and the determination of the nucleotide sequence of the coat protein (CP) (figure 2, 3 and 5), the NIa-protease gene (figure 2), the NIb-protein gene (a replicase protein) (figure 2) and the 3' untranslated region (UTR) (figure 2). In particular, the 3'-terminal 3,862 nucleotide sequence of PVBV RNA was elucidated. Analysis of the deduced amino acid sequence of the coat protein (CP) (figure 4) and nucleotide sequence of the 3'-untranslated region (3'-UTR) confirmed the identity of PVBV as a distinct potyvirus. It was furthermore found that the expressed PVBV CPs assemble into virus-like particles in E. coli. These particles were shown to encapsidate the CP-mRNA.

NIb is a replicase protein. In this protein like in other RNA polymerases GDD (i.e. glycine-aspartic acid-aspartic acid) is a conserved motif that is important for the RNA polymerase functions. In the NIb G231A mutant of the invention glycine at position 231 is substituted by alanine. By this substitution the protein looses its polymerase function but still retains its binding properties to i.e. RNA. The RNA is blocked by the binding of non-functional NIb protein to the RNA, and thus the polymerase activity, and therefore virus replication is prevented.

Based on this information the invention provides the possibility to produce transgenic plant cells and plants harbouring one or more DNA sequences being at least 80% homologous to the coat protein gene and/or the NIb-mutant protein gene, optionally in combination with the 3'-untranslated region.

Thus, the invention relates to a recombinant DNA molecule comprising one or more DNA sequences showing at least 80% homology to the coat protein gene and/or the mutant nuclear inclusion protein b (NIb) protein gene, optionally in combination with the 3'-untranslated region of a potyvirus, functionally linked with regulatory sequences needed for gene expression in plant cells.

The gene or genes comprised in the recombinant DNA molecule are in general derived from potyviridae, more in particular from the Pepper Vein Banding Virus. However, similar genes with a high level of homology (> 80%) but originating from other potyviruses are expected to give the same results in plants belonging to Capsicum spp. On the other hand, introduction of the recombinant DNA molecule according to this invention into other plants, in particular plants belonging to the Solanaceae family, is expected to result in plants that are resistant to potyviruses with at least 80% homology with PVBV.

Preferably, the recombinant DNA molecule is based on a binary vector thus facilitating Agrobacterium mediated transformation. For Agrobacterium-mediated transformation the recombinant DNA molecule is engineered into a plant expressible gene cassette, containing a reulatory elements such as a plant expressible promotor, a translation initiation codon (ATG) and a plant functional poly (A) addition signal (AATAAA). The gene cassette can be constructed in a suitable transformation vector containing the necessary sequences for obtaining expression of the recombinant molecule in plant tissue. A suitable transformation vector is for example pGA643.

The invention also relates to use of the recombinant DNA molecule for transformation, preferably for Agrobacterium mediated transformation, of plant cells and plants in order to produce plants which are resistant to the particular potyvirus of which the gene or genes is/are derived, or to other potyviruses.

In a preferred embodiment the recombinant DNA molecule of the present invention, comprises a DNA sequence that is at least 80% homologous to the 3'-terminal 3,862 nucleotide sequence of PVBV RNA as shown in figure 2. Preferably, the homology is at least 90%, more prefarably at least 95%.

The present invention also relates to plant cells which are transformed with the expression vector containing the NIb-mutant protein gene and/or coat protein gene, optionally in combination with the 3'-untranslated region, and to plant cells thus transformed which are regenerated into transgenic plants which express said genes from potyviruses.

In addition, the invention relates to a process of producing transgenic plants which are carrying the DNA sequences that are at least 80% homologous to the NIb-mutant protein gene and/or coat protein gene and/or 3'-untranslated region, which method comprises preparation of a recombinant DNA molecule carrying the above DNA sequences, transforming plant cells with the said molecule in order to obtain transgenic plant cells and regenerating complete plants out of the transgenic plant cells.

The invention further relates to seeds and progeny derived from the transgenic plants thus obtained and to a process for obtaining same.

Furthermore, the present invention relates to reproducible structures derived from a transformed plant cell or a transgenic plant resistant to PVBV, such as seeds, calluses, buds, embryos etc. obtained from the transgenic plant or the progeny derived therefrom.

Plants that can be transformed according to the invention belong for example to the family Solanaceae and more specific the species Capsicum frutescens (hot pepper), Capsicum annuum (bell- or sweet pepper) Solanum tuberosum L. (potato), Lycopersicon esculentum L. (tomato) and Solanum melongena L. (eggplant).

The invention will further be illustrated by the follwing exampls and figures, but is not limited thereto.

### BRIEF DESCRIPTION OF DRAWINGS

Figure 1 shows a plasmid map of pGA643, in which PVBV coat protein (PVBV-CP) and PVBV-CP and Non Coding Region (PVBV-CPNC) and Nib G231A (mutant) were cloned.

Figure 2 shows the nucleotide and deduced amino acid sequence of pPV-229 corresponding to the 3'-end of PVBV RNA. The predicted cleavage sites and beginning of NIa-pro, NIb and CP are indicated with arrows.
* indicates the stop codon. The trypsin-sensitive cleavage site in CP is shown by inverted filled triangle. The cleavage site residues of NIa-protease are blocked, the replicase motif in NIb is underlined.

Figure 3 shows the DNA nucleotide sequence of the CP gene of PVBV.

Figure 4 shows the amino acid sequence of PVBV CP corresponding with the nucleotide sequence of figure 3.

Figure 5 shows the linear DNA nucleotide sequence of the coat protein gene of PVBV and the corresponding restriction sites.

Figure 6 shows a SDS-PAGE analysis of the expressed PVBV CP in E. coli. The BL 21 (DE3) E. coli cells harboring PRSET-CP were induced with 0.3 mM IPTG. The CP was purified by NI-NTA agarose chromatography. Lane 1: molecular weight markers. Total proteins from uninduced (lane 2) and induced (lane 3) BL 21 (DE3) cells. Lane 4: NI-NTA affinity-purified CP.

Figure 7 represents an electron micrograph showing the expressed coat protein assembled into virus-like particles in E. coli, stained with 1 % uranyl acetate.

Figure 8 is an autoradiogram showing the slot-blot analysis of RNA isolated from assembled virus-like particles. RNA isolated from purified PVBV (panel A) and assembled virus-like particles (panel B) were spotted at different dilutions: 1:10 (lane 1), 1:100 (lane 2), 1:1000 (lane 3), 1:2000 (lane 4), 1:4000 (lane 5), 1:8000 (lane 6), 1:16000 (lane 7), 1:32000 (lane 8), and 1:64000 (lane 9) on nylon membrane and hybridized with CP-specific probe. Panel B, lane 10, represents sesbania mosaic viral RNA (1:100) as negative control.

Figure 9 shows the construction of the PVBV-CP in sense and antisense orientation in the plasmid pGA643.

Figure 10 shows the construction of the PVBV-CPNC in sense orientation in the plasmid pGA643.

Figure 11 shows the construction of the Nib G321A mutant in sense orientation in the plasmid pGA643.

### EXAMPLES

### EXAMPLE 1

### Virus purification and RNA isolation

PVBV was maintained by mechanical inoculation in Capsicum annuum cv. California Wonder under insect-proof conditions. The virions were purified as described earlier (Ravi et al., Plant Dis. 81(6), 673-676 (1997)). For RNA isolation, the virions were disrupted in dissociation buffer (0.1 M tris buffer, pH 7.5, 10 mM EDTA, 2 % SDS) followed by phenol/chloroform extraction and precipitation of RNA by isopropanol (Sambrook et al., Molecular Cloning: A laboratory manual. Cold Spring Harbor Laboratory Press, New York (1989)).

### EXAMPLE 2

### cDNA synthesis and cloning

cDNA synthesis with the isolated RNA was carried out as described earlier (Gubler and Hoffman, Gene 25, 263-269 (1983)) using Superscript (Gibco-BRL) and oligo dT primer (Perkin Elmer). The cDNA was ligated into a Hincll-cut pUC 19 vector and transformed into E. coli DH5 cells. The recombinant clones were screened for cDNA inserts by digestion with appropriate restriction enzymes.

### EXAMPLE 3

### Nucleotide sequence and analysis

Nucleotide sequencing of the cDNA clones was performed according to Sanger's dideoxy chain termination method (Sanger et al., Proc Nati Acad Sci 74, 5463-5467 (1997) manually as well as using an automated ABI Prism DNA sequencer (Perkin Elmer). In order to obtain the complete nucleotide sequence of the pPV-229 clone, exonuclease III/mungbean nuclease deletion was performed according to the procedure described by Sambrook et al (supra). The nucleotide sequence and deduced amino acid sequence were analysed using Wisconsin package.

### EXAMPLE 4

### Expression of coat protein in Escherichia coli and purification

The coat protein (CP) gene was amplified from the cDNA clone (pPV-229) using the following set of primers for cloning into expression vectors:

Sense primer: 5'AACCCGGGCATATGGCAGGAGAGAGTGTTGAT 3'
Antisense primer: 5'CTGGATCCTCACAATCCTCGAACGCC 3'

Underlined nucleotides represent the coding sequence of CP. The added nucleotides to engineer Smal and NdeI sites in sense primer and BamHII in antisense primer are represented in bold. The PCR-amplified CP gene was directly cloned at the HincII site of pUC 19 (pUC-CP). The gene fragment was released from pUC-CP by SmaI digestion and cloned in pRSET C (Invitrogen) at the Pvu Il site (pRSET-CP). The cloning strategy resulted in the addition of 41 amino acids from the pRSET C vector (including His-tag) and three amino acids (G, H and M) from the sense primer to the N-terminus of the CP sequence. The pRSET-CP was expressed in Escherichia coli (BL 21, DE3) cells by inducing with IPTG (0.3 mM). The CP was purified using Ni-NTA affinity chromatography (Qiagen Inc., Chatsworth, CA) according to the manufacturer's instructions.

### EXAMPLE 5

### Preparation of DNA probe and RNA slot-blot analysis

The CP fragment was released from pRSET-CP by BamHI digestion and was used for random primer labelling as described by Sambrook et al. (supra). RNA was isolated from the assembled coat proteins as described earlier for the isolation of PVBV RNA and was serially diluted in 1x SSC and blotted onto HybondN-N membrane (Pharmacia-Amersham, Biotech) using Hybri-slot Manifold (Gibco-BRL). The membrane was baked for two hours at 80°C.
Prehybridization was carried out in 50 % (v/v) formamide, 10 % (w/v) dextran sulphate, 1 % (w/v) SDS, 0.9 M NACI and 200 µg/ml salmon sperm DNA for 6 hours at 42°C. Hybridization was performed as described by Sambrook et al (supra). The membranes were washed with 2x SSC, 0. 1 % SDS, 1x SSC, 0. 1 % SDS, 0.5x SSC, and 0.1 % SDS at 60 °C and exposed for autoradiography.

### EXAMPLE 6

### cDNA synthesis and sequencing of the cDNA clones

Among the cDNA clones obtained, the pPV-229 clone had the maximum size insert of about 3.9 kb. pPV-229 and its exonuclease III deletion clones were sequenced. The 3'-terminal 3,862 nt genomic sequence of PVBV is shown in Fig. 2.

The analysis of the nucleotide sequence showed a single partial ORF encoding a partial VPg domain, Nla protease domain, Nlb, CP and 3'-UTR. The Nla cleavage sites between NIa-pro/Nlb and sub-optimal cleavage site within the Nla protein (VPg/NIa-pro) were predicted based on the similarity of amino acid sequence at the corresponding cleavage sites in other potyviruses. The cleavage site between Nlb/CP has been confirmed as N-terminal amino acid sequence of the intact CP which was determined earlier by automated sequencing (Ravi et al., Plant Dis. 81 (6), 673-676 (1997)).

The 3'-UTR was 280 nucleotides in length and had a poly A tail at the 3'-end as observed in other potyviruses (Richmann et al., J Gen Virol. 73, 1-16 (1992)). The 3'-UTR showed no significant similarity to any of the known potyviral 3'-UTR sequences. The amino acid sequence comparison of PVBV proteins with those of other five potyviruses with maximum identity is shown in Table 1.

The amino acid sequence similarity of CP with other potyviral CPs and nucleotide sequence similarity of 3'-UTR of viral RNA have been considered as criteria for classification of potyviruses (Shukla et al., Adv Virus Res. 36, 273-314 (1989); Frenkel et al., J gen Virol. 70, 2775-2783 (1989)). As the PVBV CP showed a maximum amino acid sequence similarity with plum pox virus (only 70 % identity), and as there is no significant sequence similarity of PVBV 3'-UTR with that of other known potyviruses, the earlier observation that PVBV is a distinct potyvirus infecting pepper is confirmed (Ravi et al., supra).

The coat protein has 50 amino acids to the N-terminus of a trypsin cleavage site. The N-terminal amino sequence has no significant similarity with N-termini of other potyviral CPs, whereas the C-terminal region is highly conserved.

The PVBV NIb protein showed maximum similarity to that of PPV (table 1). It had the conserved GDD motif, the typical signature motif present in RNA-dependent RNA polymerases of RNA viruses.

The Nla-protease domain showed a marginally higher similarity to TuMV than PPV Nla-protease. From the analysis it was evident that, of the three proteins, Nla-protease is less conserved among potyviruses. However, the catalytic residues (blocked in Fig 2) are conserved in PVBV Nla-protase as in other potyviruses (Richmann et al., supra). Overall, PVBV is closest to PPV.

**Table 1.**

| Percent identity of amino acid sequence of PVBV proteins with those of other potyviruses. | | | |
|---|---|---|---|
| | NIa-pro | Nib | CP |
| PPV | 48.76 | 62.21 | 70.38 |
| TuMV | 50.00 | 61.85 | 65.61 |
| TEV | 47.11 | 60.20 | 65.91 |
| PVY | 48.35 | 59.50 | 59.22 |
| PeMV | 48.35 | 59.84 | 60.28 |
| PPV= Plum Pox Virus; TuMV=Turnip Mosaic Potyvirus; TEV= Tobacco etch Virus; PVY=Potato Virus Y; PEMV= Pepper Mottle Virus. Comparison was mede using GCG package 9version 9.1). Sequence data: PPV: Maiss et al., J Gen Virol. 70, 513-524 (1989); TuMV: Nicolas et al., J gen Virol. 73, 2785-2793 (1992); TEV: Allison et al., Virology 154, 9-20 (1986); PVY: Robaglia et al., J Gen Virol 70, 935-947 (1989); PeMV: Vance et al., Virology 191, 19-30 (1992). | | | |

### EXAMPLE 7

### Characterization of PVBV expressed in Escherichia Coli

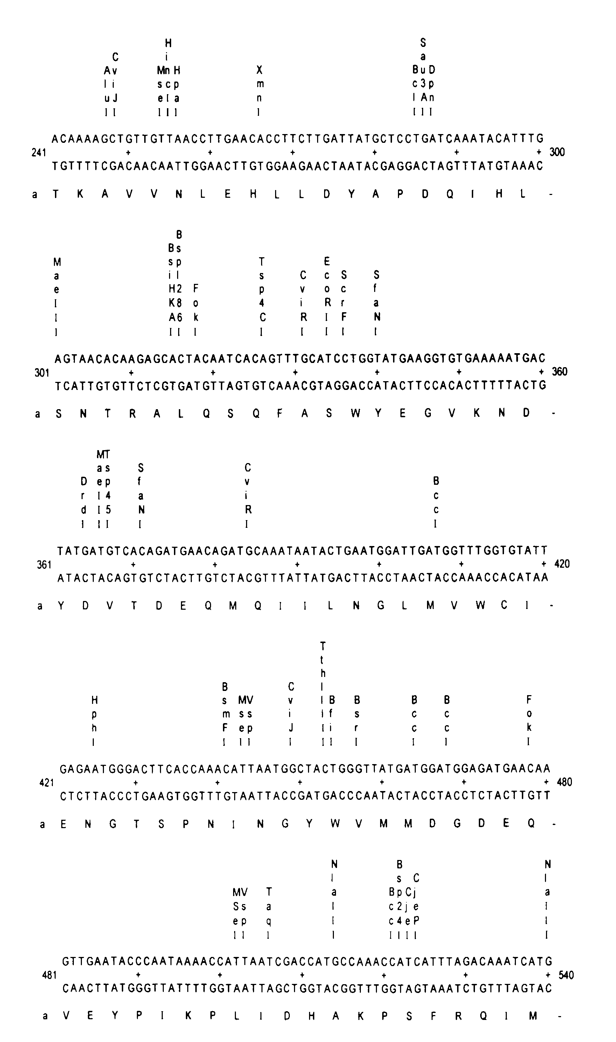

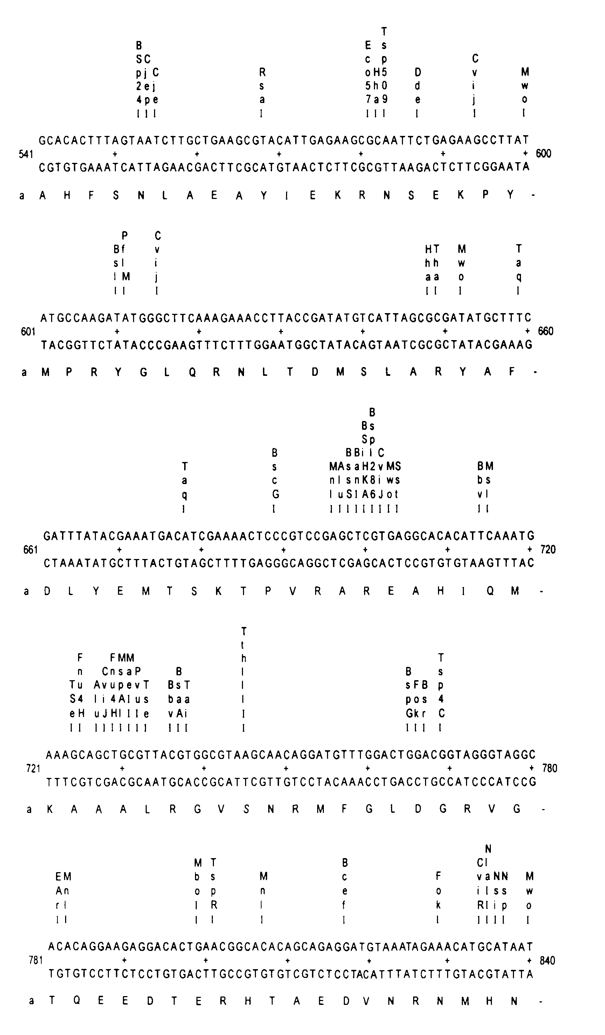

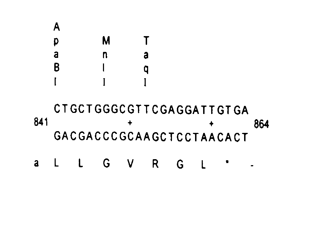

The CP expressed in E. coli was purified by Ni-NTA affinity chromatography as mentioned in example 1. Results of SDS-PAGE analysis are shown in figure 6. Electron microscopic studies revealed that the expressed PVBV CP assembled into virus-like particles in E. coli (Fig. 7) as reported earlier for Johansongrass Mosaic Virus (Jagadish et al., J Gen Virol. 72, 1543-1550 (1991)). The particles were heterogeneous in length as compared to purified PVBV. Removal of the additional amino acids resulting from the cloning strategy at the N-terminus of expressed CP did not have any effect on the assembly process (data not shown).

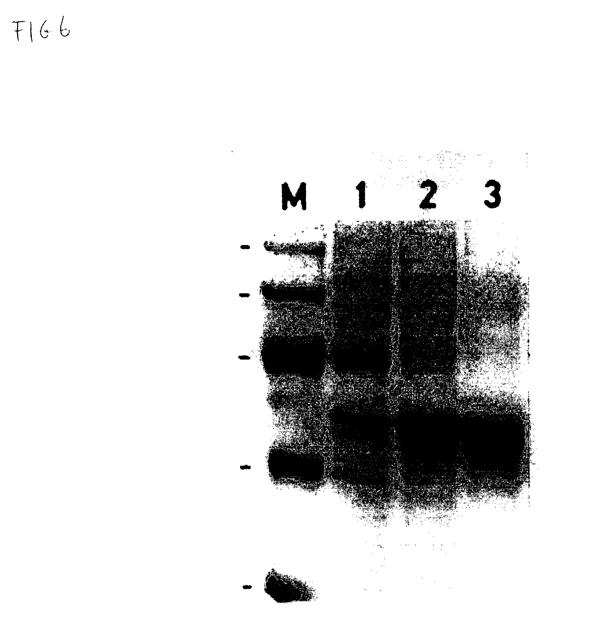

Furthermore, in order to check if the assembled particles encapsidated the CP mRNA from E. coli, RNA was isolated from the virus-like particles and slot blot hybridization was carried out with a PVBV CP-specific probe. The result showed that the assembled virus-like particles encapsidated its own messenger RNA (Fig 8). Although encapsidation of mRNA has been demonstrated for the recombinant capsids of spherical viruses (Agarwal et al., Virology 207, 89-97 (1995)), to our knowledge this is the first report of encapsidation of CP mRNA by recombinant CPs of flexuous rod-shaped viruses.

The nucleotide sequence required for the nucleation of assembly may lie within the RNA region encoding the CP, or the mRNA encapsidation may indeed be non-specific. The assembly in potyviruses has been shown to begin from 5'-end of the RNA (Wu et al., J Gen Virol. 79, 1525-1529 (1998)). However, possibly, in the absence of 5'-end of the sequence, the 3'-end (CP region) can act as nucleation signal for the virus assembly. The length of the assembled particles is variable suggesting that mRNA encapsidation is not limited by the length of the particles and the RNA may only act as nucleation centre for the assembly. Also, the addition of 44 amino acids at the N-terminus of the CP (due to the cloning strategy) did not affect the assembly of CP into virus-like particles. This is in concordance with results showing that expression of foreign epitopes at the N-terminus of potyviral CPs did not affect the assembly (Jagadish et al., Bio/Technology 11, 1166-1170 (1993)).

### EXAMPLE 8:

### Construction of a PVBV Coat Protein- ,a NIb-mutant protein- and Non Coding Region- Gene Cassette for expression in plants

### CLONING OF PVBV-CP IN pGA643

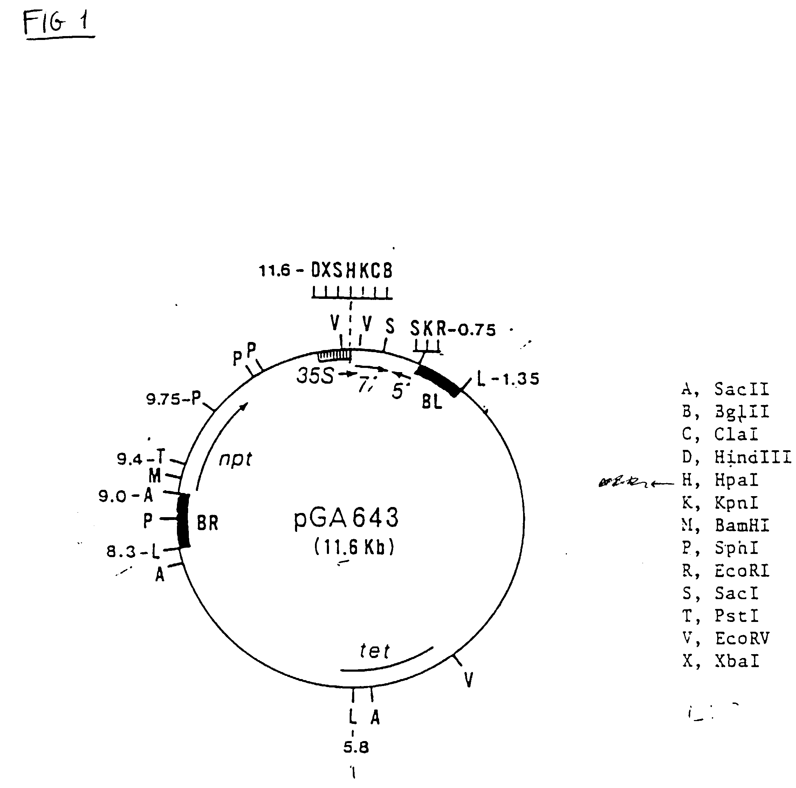

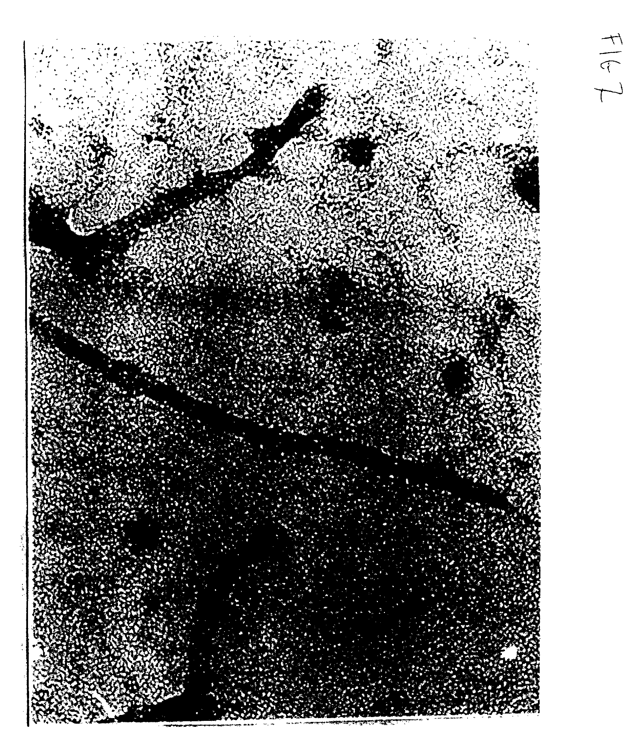

The coat protein of PVBV in pRSET-CP was released by BamH 1 digestion and cloned into pGA643 at Bgl II site (figure 1). Digestion with Ndel /EcoR I confirmed its orientation (figure 9).

### CLONING OF PVBV-CPNC IN pGA643

The coat protein/ 3'-non coding region (CPNC) was amplified from the cDNA clone (pPV-229) using the the following pair of primers for cloning into pUC 19:

CP sense 5' AACCCGGGCATATGGCAGGAGAGAGAGTTGAT 3'
NC antisense 5' TTGGATCCTTTAACGCCACTATTGAA 3'

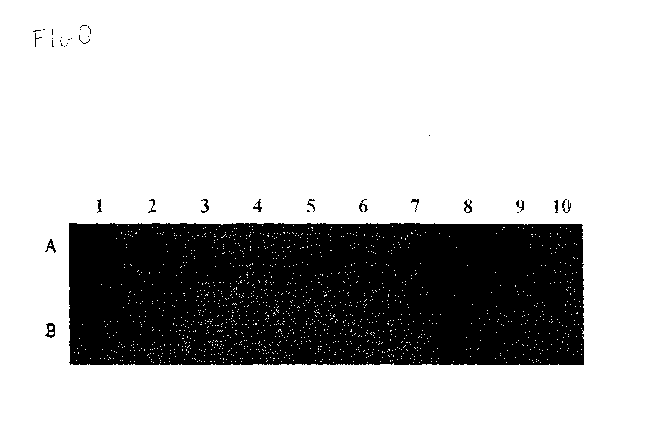

The PCR-amplified CPNC fragment was cloned at the Hinc II site of pUC19 (pUC-CPNC). The gene fragment was released from pUC-CPNC by Hind III/ BamH I digestion and cloned in pGA643 at Hind III/ Bgl II site. Digestion with Hind III/ EcoR I confirmed its presence (figure 10).

### CLONING OF PVBV-NIb-MUTANT IN pGA643

The NIb region was amplified from the cDNA clone (pPV-229) using the following pair of primers for cloning into pUC 19:

Sense 5' GTTCCCGGGATGGCAAGTGATAAATGGCTG 3'
Anti sense 5' TCACTGATGGGCAACTTCACCGCC 3'

The PCR amplified fragment was cloned at the Hinc II site of pUC 19. The insert was released from pUC 19 using Sma I/BamH I digestion and cloned into pGA643 at Hpa I and Bgl II sites. Digestion with Hind III confirmed the presence of a 1.2 Kb fragment.

### Mutation in the GDD-motif:

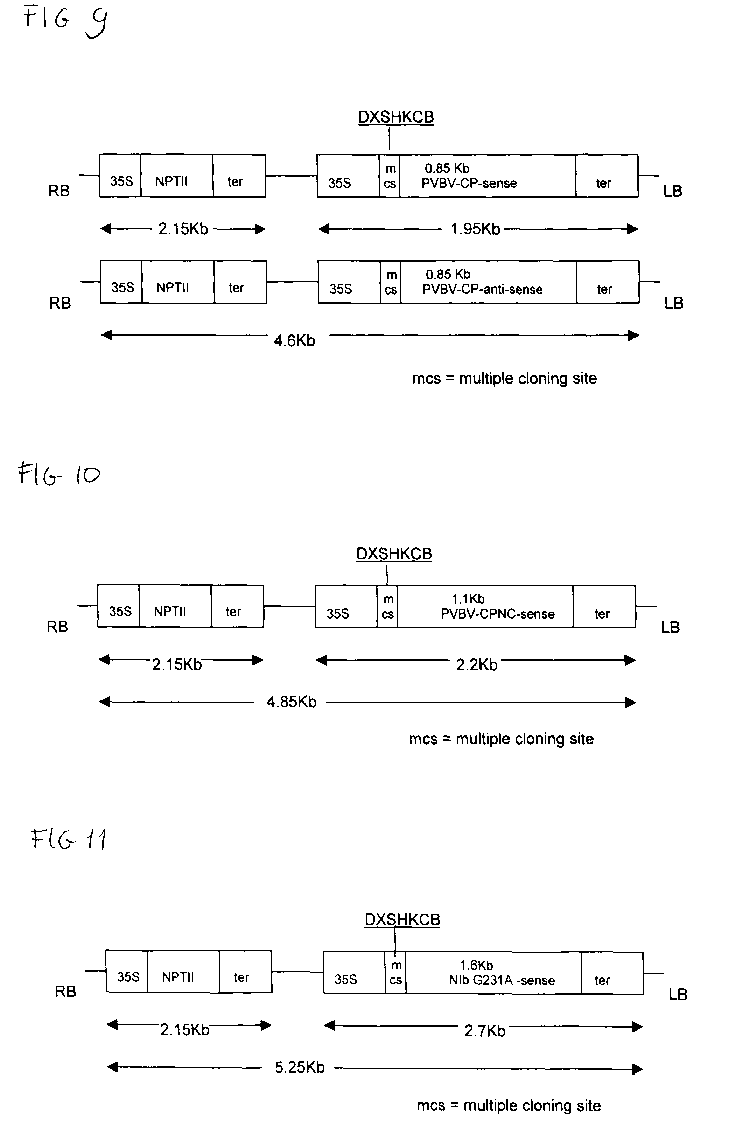

NIb G231A mutation was introduced using the following primer 5' GTTTTCTTCGCGAATGCTGATGAC 3'. The underlined sequence corresponds to a Nru I site and is used for screening purposes. Mutation was introduced by using a mega primer method using Pfu DNA polymerase from "Statagene", according to the manufactures instructions. Mutants were screened by digestion with Nru I and sequence was confirmed by direct DNA sequencing (figure 11) .

### EXAMPLE 9

### Agrobacterium mediated transfer of PVBV Coat Protein Gene Cassette into Plant Tissues

Agrobacterium tumefaciens mediated transformation was used to introduce the PVBV Coat Protein gene, the NIb G231A gene and the Non Coding Region (NCR), single or in any combination, into plant tissues of several crops, preferably in Capsicum frutescens and Capsicum annuum.

Seeds of Capsicum frutescens breeding lines A, B and C and seeds of Capsicum annuum breeding lines D and E were germinated on 1/2 MS medium, kept in the dark for four days and subsequently shifted to light. Hypocotyl and cotyledon explants were taken from 8 to 20 day old seedlings and pre-incubated on 0.5 mg per liter IAA + 2.0 mg per liter BAP for two days.

A strain of Agrobacterium tumefaciens, containing a vector carrying the PVBV Coat Protein gene in sense orientation, carrying the PVBV Coat Protein in anti-sense orientation, carrying the PVBV Coat Protein in combination with the non coding region in sense orientation, carrying the NIb G231A gene in sense orientation and linked to the selectable marker gene NPTII and the CaMV 35S promotor, was cultured overnight. The pre-incubated hypocotyl and cotyledon explants were incubated for a period of 10 to 20 minutes in suspension of the Agrobacterium tumefaciens strain in MS Liquid Medium with a O.D. of between 0.1 and 0.8.

The explants were cocultivated for two days under dark conditions and transferred to regeneration medium, containing 25-100 mg per liter of the antibiotic kanamycin. Every 8 to 20 days explants were transferred to fresh regeneration medium. The regeneration medium consisted of MS medium + 0.2 to 1.5 mg per liter IAA + 1.0 to 5.0 mg per liter BAP.

Shoot buds were formed especially from cut ends of the explants between 4 and 15 weeks after cocultivation. These shoot buds were transferred to MS medium + 0.5 to 2.0 mg per liter IAA + 1.0 to 5.0 mg per liter BAP + 0.5 to 2.0 mg per liter GA + 2.0 to 10.0 mg per liter AgNO3. At this medium elongation of the shoots took place within 15 days. The elongated shoots were transferred to rooting media containing MS + 0.5 to 2.0 mg per liter IBA. Rooted shoots were transferred to peat soil and transferred to the greenhouse.

### EXAMPLE 10

### Improved resistance to PVBV (Pepper Vein Banding Virus) in transgenic Capsicum plants

Transformed plants of Capsicum frutescens variety C and Capsicum annuum variety D containing PVBV-CP-sense, PVBV-CP-anti sense, PVBV-CPNC-sense and PVBV-NIb G231A-sense were transferred from "in vitro" to peat soil in the greenhouse. To test resistance of the transgenic plants against the Pepper Vein Banding Virus, one transgenic plant of each variety-gene combination was used for artificial inoculation with a virus culture.

Cultures of Pepper Vein Banding Virus were maintained on a susceptible Capsicum variety under laboratory conditions. When the fifth leaf of the plants was fully extended, leaf tissue from the infected plants was ground in a mortar. Sap was strained through butter muslin and diluted ten times with distilled water. The transgenic plants and non-transgenic control plants of the same variety were dusted with a fine abrasive powder and each plant was inoculated with infected sap, which is applied to the leaf surfaces by gently smoothing with finger and thumb. Directly after inoculation the plants are lightly watered to avoid surface oxidation.

On susceptible plants symptoms of virus infection appeared already after about five days resulting in a typical mottling and dwarf growth after fourteen days. For scoring the level of resistance, a scale from 0 to 9 was used, in which 0 corresponds to very susceptible plants showing severe symptoms, while 9 corresponds to very resistant plants showing no symptoms. Results of the test on the level of resistance are shown in table 3. Control plants of variety C showed a resistance level of 2, while variety D did not show any resistance, resulting in a score of 0. Transformed plants of Capsicum frutescens variety C and Capsicum annuum variety D containing PVBV-CP-sense, PVBV-CP-anti sense, PVBV-CPNC-sense and PVBV-NIb G231A-sense all showed an increased level of resistance, varying from score 4 to 8. The plants containing at least the coat protein (CP) gene showed a significant increase in resistance to PVBV.

**Table 2.**

| | | |
|---|---|---|
| Level of PVBV tolerance of pepper plants of variety C and D after transformation with a gene construct containing CP-sense, CP-antisense, CPNC-sense and NIb G231A-sense. As a control the wild type plants of variety C and D were used. | | |

| | Variety C | variety D |
|---|---|---|
| CP sense | 8 | 8 |
| CP antisense | 6 | 7 |
| CPNC sense | 8 | 8 |
| NIb G231A sense | 5 | 4 |
| Control | 2 | 0 |

### ABBREVIATIONS

- DNA: deoxyribonucleic acid
- RNA: ribonucleic acid
- PVBV: pepper vein banding virus
- ORF: open reading frame
- VPg: viral encoded genome linked protein
- NIa-pro: nuclear inclusion protein a (protease)
- NIb-pro: nuclear inclusion protein b
- NIb G231A: mutated NIb in which the Glycine 231 has been changed to Alanine
- CP: coat protein
- UTR: untranslated region
- NCR: non coding region
- CPNC: coat protein / 3'non coding region
- EDTA: ethylenediaminetetra acetic acid
- SDS: sodium dodecyl sulfate
- Oligo dT primer (18mer): deoxy thymidine polynucleotide
- IPTG: isopropyl-beta-D-thiogalactopyranoside
- Ni-NTA: nickel-nitrilotriacetic
- SSC: saline (150mM) sodium citrate (15mM), pH7
- GGD motif: glycine aspartic acid aspartic acid motif
- MS: Murashige & Skoog
- BAP: 6-benzyl amino purine
- NPTII: neomycin phosphotranspherase II
- CaMV: cauliflower mosaic virus
- O.D.: optical density
- IAA: indole acetic acid
- GA: gibberellic acid
- AgNO3: silver nitrate
- IBA: indole butric acid
- PPV: plum pox virus
- TuMV: turnipmosaic virus
- TEV: tobacco etch potyvirus
- PVY: potato Y potyvirus
- PeMV: pepper mottle virus
- MCS: multiple cloning site

## Claims

1. A recombinant DNA molecule comprising one or more DNA sequences showing at least 80% homology to the coat protein gene and/or the mutant nuclear inclusion protein b (NIb) protein gene optionally in combination with the 3'-untranslated region of a potyvirus, functionally linked with regulatory sequences needed for gene expression in plant cells.

2. A recombinant DNA molecule according to claim 1 wherein the potyvirus is Pepper Vein Banding Virus (PVBV).

3. A recombinant DNA molecule according to claim 1 or 2 **characterized in that** the DNA sequences show at least 80% homology to the sequence as shown in figure 2.

4. A recombinant DNA molecule according to claim 3 **characterized in that** sequences show at least 90% homology to the sequence as shown in figure 2.

5. A recombinant DNA molecule according to claim 3 **characterized in that** sequences show at least 95% homology to the sequence as shown in figure 2.

6. A transformed plant cell comprising a recombinant DNA molecule as claimed in claims 1-5.

7. A transgenic plant, belonging to the family Solanaceae and more specific the species Capsicum frutescens (hot pepper), Capsicum annuum (bell- or sweet pepper) Solanum tuberosum L. (potato), Lycopersicon esculentum L. (tomato) and Solanum melongena L. (eggplant) comprising transformed plant cells according to claim 6.

8. Seeds of a transgenic plant comprising transformed plant cells according to claim 6.

9. Seeds of a transgenic plant, belonging to the family Solanaceae and more specific the species Capsicum frutescens (hot pepper), Capsicum annuum (bell- or sweet pepper) Solanum tuberosum L. (potato), Lycopersicon esculentum L. (tomato) and Solanum melongena L. (eggplant) comprising transformed plant cells according to claim 6.

10. A transgenic plant, belonging to the genus Capsicum, comprising of transformed plant cells according to claim 6.

11. A transgenic plant according to claim 10, belonging to the species Capsicum frutescens and Capsicum annuum.

12. Seeds of a transgenic plant, belonging to the genus Capsicum, comprising transformed plant cells according to claim 6.

13. Seeds of a transgenic plant, belonging to the species Capsicum frutescens and Capsicum annuum, comprising transformed plant cells according to claim 6.

14. A process for producing a transgenic plant which is resistant to viral infection, which method comprises of transforming plant cells with a recombinant DNA-molecule according to any of the claims 1-5 and regenerating plants from the transformed plant cells.


### Annex to the description

SEQUENCE LISTING

<110> Bejo Zaden B.V.

<120> Coat protein genes of potyvirus of Capsicum spp. and
      plants transformed therewith

<130> bejo4

<140> 99200261.8
<141> 1999-01-29

<160> 13

<170> PatentIn Ver. 2.1

<210> 1
<211> 32
<212> DNA
<213> Artificial Sequence

<220>
<223> Description of Artificial Sequence:sense primer

<400> 1
aacccgggca tatggcagga gagagtgttg at                                32


<210> 2
<211> 26
<212> DNA
<213> Artificial Sequence

<220>
<223> Description of Artificial Sequence:antisense
      primer

<400> 2
ctggatcctc acaatcctcg aacgcc                                       26


<210> 3
<211> 32
<212> DNA
<213> Artificial Sequence

<220>
<223> Description of Artificial Sequence:primer

<400> 3
aacccgggca tatggcagga gagagagttg at                                32

<210> 4
<211> 26
<212> DNA
<213> Artificial Sequence

<220>
<223> Description of Artificial Sequence:primer

<400> 4
ttggatcctt taacgccact attgaa                                       26

<210> 5
<211> 30
<212> DNA
<213> Artificial Sequence

<220>
<223> Description of Artificial Sequence:antisense
      primer

<400> 5
gttcccggga tggcaagtga taaatggctg                                   30

<210> 6
<211> 24
<212> DNA
<213> Artificial Sequence

<220>
<223> Description of Artificial Sequence:antisense
      primer

<400> 6
tcactgatgg gcaacttcac cgcc                                         24

<210> 7
<211> 24
<212> DNA
<213> Artificial Sequence

<220>
<223> Description of Artificial Sequence: primer

<400> 7
gttttcttcg cgaatgctga tgac                                              24



<210> 8
<211> 3862
<212> DNA
<213> pepper vein banding virus

<220>
<221> CDS
<222> (1)..(3579)

<400> 8
tca aaa ggg aag aca cat ggg atg ggg aag aag aat agg agg ttc gtg    48
Ser Lys Gly Lys Thr His Gly Met Gly Lys Lys Asn Arg Arg Phe Val
  1               5                  10                  15

aac atg tac ggt ttc gat cca acc gag tat tca ttt att cgg ttc gtc    96
Asn Met Tyr Gly Phe Asp Pro Thr Glu Tyr Ser Phe Ile Arg Phe Val
             20                  25                  30

gat ccg gtt aca ggt gaa atg ctt gat gaa agt gtc atg gca gat gta   144
Asp Pro Val Thr Gly Glu Met Leu Asp Glu Ser Val Met Ala Asp Val
         35                  40                  45

atg cta gta cag gaa cac ttc aat gac ctg cgc ttt gaa tat ttg gga   192
Met Leu Val Gln Glu His Phe Asn Asp Leu Arg Phe Glu Tyr Leu Gly
     50                  55                  60

gat gat aaa att gaa agt gcc aga cta tac agc aat cct gga att caa   240
Asp Asp Lys Ile Glu Ser Ala Arg Leu Tyr Ser Asn Pro Gly Ile Gln
 65                  70                  75                  80

gca tac ttt gtc aaa gac aag gtc tct cct gtt ctg aaa gtc gat ctt   288
Ala Tyr Phe Val Lys Asp Lys Val Ser Pro Val Leu Lys Val Asp Leu
                 85                  90                  95

aca caa cat gta cct ctt aaa gtg tgt gac aac tca tca acg ata gct   336
Thr Gln His Val Pro Leu Lys Val Cys Asp Asn Ser Ser Thr Ile Ala
            100                 105                 110

ggg ttt ccg gaa aga gaa ggt gag ttc cga caa acc ggt caa gcc aca   384
Gly Phe Pro Glu Arg Glu Gly Glu Phe Arg Gln Thr Gly Gln Ala Thr
        115                 120                 125

aag gtc aac tat gat gag ctt cca tca aga gaa gga gtt gag cat gaa 432
Lys Val Asn Tyr Asp Glu Leu Pro Ser Arg Glu Gly Val Glu His Glu
    130                 135                 140

gcc cgc tcg tta aat aga ggg ctc cgg gat cac aac caa gta tcg aag 480
Ala Arg Ser Leu Asn Arg Gly Leu Arg Asp His Asn Gln Val Ser Lys
145                 150                 155                 160

ttg att tgc aag ttg gaa aat gat tcc gca tga ttg tgt tac ctc aat 528
Leu Ile Cys Lys Leu Glu Asn Asp Ser Ala Leu Cys Tyr Leu Asn
                165                 170                 175

tca tgg tgt tgg ctt cgg gtc cat tat aat tac aaa tcg tca ttt act 576
Ser Trp Cys Trp Leu Arg Val His Tyr Asn Tyr Lys Ser Ser Phe Thr
                180                 185                 190

caa aag gaa caa tga acc ctt cga gtc aaa aca gca cat ggc gac ttc 624
Gln Lys Glu Gln Thr Leu Arg Val Lys Thr Ala His Gly Asp Phe
            195                 200                 205

aaa gtg gct aac aca aag gag atg aag gtg ttc ccc gta gag aaa cat 672
Lys Val Ala Asn Thr Lys Glu Met Lys Val Phe Pro Val Glu Lys His
            210                 215                 220

gac att ctg ctc att agg ctt cca aag gat ttc cca cca ttt cca gtg 720
Asp Ile Leu Leu Ile Arg Leu Pro Lys Asp Phe Pro Pro Phe Pro Val
225 230 235 240

aag agt aag ttt aga gag ccc aag gtc aat gat tca att tgc tta gtt 768
Lys Ser Lys Phe Arg Glu Pro Lys Val Asn Asp Ser Ile Cys Leu Val
245 250 255

ggc aca aac ttt caa gag aag ttt ttg agt agt ctt att tca gca gat 816
Gly Thr Asn Phe Gln Glu Lys Phe Leu Ser Ser Leu Ile Ser Ala Asp
260 265 270

agc aca aca tca cca gtg agt gga tca aag ttt tgg aga cac tgg att 864
Ser Thr Thr Ser Pro Val Ser Gly Ser Lys Phe Trp Arg His Trp Ile
                275                 280                 285

gat aca aag gat gga cac tgt ggc ttg cca tta gta gct cga gat gat 912
Asp Thr Lys Asp Gly His Cys Gly Leu Pro Leu Val Ala Arg Asp Asp
            290                 295                 300

ggt gca att gtt ggg ttt cac agt ctc aca agt atc aac aca gaa caa 960
Gly Ala Ile Val Gly Phe His Ser Leu Thr Ser Ile Asn Thr Glu Gln
305 310 315 320

aac tac ttt gca gct gtt cca gaa gct ttc atg gaa tta att gcc cag 1008
Asn Tyr Phe Ala Ala Val Pro Glu Ala Phe Met Glu Leu Ile Ala Gln
325 330 335

gtg gag act tta gag tgg cgg aaa agt tgg gtg tac aat cca aat gaa 1056
Val Glu Thr Leu Glu Trp Arg Lys Ser Trp Val Tyr Asn Pro Asn Glu
340 345 350

ata gga tgg ggc tcc ttg aaa ttg aag agc gat caa ccg act gga atg 1104
Ile Gly Trp Gly Ser Leu Lys Leu Lys Ser Asp Gln Pro Thr Gly Met
                355                 360                 365

ttt aag att gaa aaa ctc att gag gat atc cag tca gca ttt gtt cgt 1152
Phe Lys Ile Glu Lys Leu Ile Glu Asp Ile Gln Ser Ala Phe Val Arg
            370                 375                 380

gag caa gca agt gat aaa tgg ctg tat gca cag ttt caa ggg ttt ctc 1200
Glu Gln Ala Ser Asp Lys Trp Leu Tyr Ala Gln Phe Gln Gly Phe Leu
385 390 395 400

aaa gcc gtg cca aag agt gag agc caa ctt gta ccc aag cac att gtt 1248
Lys Ala Val Pro Lys Ser Glu Ser Gln Leu Val Pro Lys His Ile Val
405 410 415

aag gga cca tgc ccg ttg ttt cat ctt tat ctc agc aca cat cgt gag 1296
Lys Gly Pro Cys Pro Leu Phe His Leu Tyr Leu Ser Thr His Arg Glu
420 425 430

gca aga caa ttt ttc caa ccc ttc atg ggt gcg tat ggt aag agc cga 1344
Ala Arg Gln Phe Phe Gln Pro Phe Met Gly Ala Tyr Gly Lys Ser Arg
                435                 440                 445

ttg aat cgt att gcc tat gct aag gac att ttg aag tat tcc act gaa 1392
Leu Asn Arg Ile Ala Tyr Ala Lys Asp Ile Leu Lys Tyr Ser Thr Glu
            450                 455                 460

ata gaa gtt ggg aag gtt gat aca tgt gca ctt gaa ctg ccc gtt gac 1440
Ile Glu Val Gly Lys Val Asp Thr Cys Ala Leu Glu Leu Pro Val Asp
465 470 475 480

gac gta att gca gac ttg cat gct gtc aaa ttt gat act tgc aat tat 1488
Asp Val Ile Ala Asp Leu His Ala Val Lys Phe Asp Thr Cys Asn Tyr
485 490 495

gtc act gat gaa gaa gag att ttc caa tct ctt aac atg aaa gca gct 1536
Val Thr Asp Glu Glu Glu Ile Phe Gln Ser Leu Asn Met Lys Ala Ala
500 505 510

gtt ggt gca ttg tat aag ggt aag aaa cgc gag tat ttt gaa aca tac    1584
Val Gly Ala Leu Tyr Lys Gly Lys Lys Arg Glu Tyr Phe Glu Thr Tyr
        515                 520                 525

acc caa cag gac aaa gag cga ata ctg ttt gaa agt tgt ctt aga ctg    1632
Thr Gln Gln Asp Lys Glu Arg Ile Leu Phe Glu Ser Cys Leu Arg Leu
    530                 535                 540

tat aaa gga aac ctt gga gtg tgg aat ggt tca atc aag gct gag ctt    1680
Tyr Lys Gly Asn Leu Gly Val Trp Asn Gly Ser Ile Lys Ala Glu Leu
545                 550                 555                 560

aga cct att gag aaa gtg cgt gct aac aag acg cga act ttt aca gca    1728
Arg Pro Ile Glu Lys Val Arg Ala Asn Lys Thr Arg Thr Phe Thr Ala
                565                 570                 575

gca cca ttg gat acg cta cta gct ggc aaa gtt tgc gtt gat gat ttc    1776
Ala Pro Leu Asp Thr Leu Leu Ala Gly Lys Val Cys Val Asp Asp Phe
            580                 585                 590

aac aac cag ttc tac tca aag cac aca ctt gca cca tgg agt gtc gga    1824
Asn Asn Gln Phe Tyr Ser Lys His Thr Leu Ala Pro Trp Ser Val Gly
        595                 600                 605

att tca aaa ttc tcg ggt ggt tgg gat aga ttg ttg agg aaa cta cct    1872
Ile Ser Lys Phe Ser Gly Gly Trp Asp Arg Leu Leu Arg Lys Leu Pro
    610                 615                 620

gac gga tgg ata tac tgt agc gca gat ggc agt aga ttt gat agt tct    1920
Asp Gly Trp Ile Tyr Cys Ser Ala Asp Gly Ser Arg Phe Asp Ser Ser
625                 630                 635                 640

tta aca cca tat ctc ata aat gct gtg gcg tcc atc cgt ctg aaa ttc    1968
Leu Thr Pro Tyr Leu Ile Asn Ala Val Ala Ser Ile Arg Leu Lys Phe
                645                 650                 655

atg gaa ccg tgg gga atc ggg gag caa atg cta atg agg aac ttg tat    2016
Met Glu Pro Trp Gly Ile Gly Glu Gln Met Leu Met Arg Asn Leu Tyr
            660                 665                 670

gcc gaa atc ata tat act ccc att ctc act gct gat gga acg ata gtt    2064
Ala Glu Ile Ile Tyr Thr Pro Ile Leu Thr Ala Asp Gly Thr Ile Val
        675                 680                 685

aag aag tcc aaa ggc aat aac tcg gga caa cca tcg aca gtc gtg gat    2112
Lys Lys Ser Lys Gly Asn Asn Ser Gly Gln Pro Ser Thr Val Val Asp
    690                 695                 700

aac aca ctc atg gtg tta gtg gca atg aag tac tca cta cgt cgt ctg 2160
Asn Thr Leu Met Val Leu Val Ala Met Lys Tyr Ser Leu Arg Arg Leu
705 710 715 720

ggt att att ata agg atc aaa aac gac agg ggc gtt ttc ttt gcc aat 2208
Gly Ile Ile Ile Arg Ile Lys Asn Asp Arg Gly Val Phe Phe Ala Asn
725 730 735

ggt gat gac tta att gtc tca gtt ccg cca agt gat gaa tgg att ttg 2256
Gly Asp Asp Leu Ile Val Ser Val Pro Pro Ser Asp Glu Trp Ile Leu
740 745 750

gac tcg ttg cag gac cgt tct tca gag cta ggc ttg tca tat gat ttt 2304
Asp Ser Leu Gln Asp Arg Ser Ser Glu Leu Gly Leu Ser Tyr Asp Phe
                755                 760                 765

aat gaa cga aca aca gat aga tca gag ctt tgg ttt atg tcc cat caa 2352
Asn Glu Arg Thr Thr Asp Arg Ser Glu Leu Trp Phe Met Ser His Gln
            770                 775                 780

gga att tta att gag aat caa tat atc cca aag ctt gag ccc gag cgc 2400
Gly Ile Leu Ile Glu Asn Gln Tyr Ile Pro Lys Leu Glu Pro Glu Arg
785 790 795 800

att gtt tca att ctt gag tgg gat aga gct gag cag cca gag cat cga 2448
Ile Val Ser Ile Leu Glu Trp Asp Arg Ala Glu Gln Pro Glu His Arg
805 810 815

ctt gaa gcc att tgt gca tct atg att gaa gca tgg ggt cac aag gaa 2496
Leu Glu Ala Ile Cys Ala Ser Met Ile Glu Ala Trp Gly His Lys Glu
820 825 830

tta ttg tat gag ata cga ttg ttc tac aaa tgg gtc att gaa caa gca 2544
Leu Leu Tyr Glu Ile Arg Leu Phe Tyr Lys Trp Val Ile Glu Gln Ala
                835                 840                 845

ccg tac tcg cag atc gta tct gaa ggg aaa gca cca tac att tca gag 2592
Pro Tyr Ser Gln Ile Val Ser Glu Gly Lys Ala Pro Tyr Ile Ser Glu
            850                 855                 860

aca gca ttg agg tgc ctt aat atg agc gaa cat gga gaa aat gat att 2640
Thr Ala Leu Arg Cys Leu Asn Met Ser Glu His Gly Glu Asn Asp Ile
865 870 875 880

aat cca tac ttg aga gcg ctc att gag gga gct aag cgg gaa gaa ttg 2688
Asn Pro Tyr Leu Arg Ala Leu Ile Glu Gly Ala Lys Arg Glu Glu Leu
885 890 895

gat gat gat ggc ggt gaa gtt gcc cat cag gca gga gag agt gtt gat 2736
Asp Asp Asp Gly Gly Glu Val Ala His Gln Ala Gly Glu Ser Val Asp
900 905 910

gct ggg agc gta aaa ggc gaa gac tca tca agc aaa tca gct gat aaa 2784
Ala Gly Ser Val Lys Gly Glu Asp Ser Ser Ser Lys Ser Ala Asp Lys
                915                 920                 925

caa gcc aca gag aaa aag aac aaa gtg agt gga caa gct cag cca cag 2832
Gln Ala Thr Glu Lys Lys Asn Lys Val Ser Gly Gln Ala Gln Pro Gln
            930                 935                 940

tct cgt cag agt gaa atg gaa gta ccc caa gtt cga gac aga gat gtt 2880
Ser Arg Gln Ser Glu Met Glu Val Pro Gln Val Arg Asp Arg Asp Val
945 950 955 960

aac gtt ggg act tca gga acg ttc aca ata ccg cgt ctt aag ggt att 2928
Asn Val Gly Thr Ser Gly Thr Phe Thr Ile Pro Arg Leu Lys Gly Ile
965 970 975

ttt tca aag tta aca ata cca aag gtc aaa aca aaa gct gtt gtt aac 2976
Phe Ser Lys Leu Thr Ile Pro Lys Val Lys Thr Lys Ala Val Val Asn
980 985 990

ctt gaa cac ctt ctt gat tat gct cct gat caa ata cat ttg agt aac 3024
Leu Glu His Leu Leu Asp Tyr Ala Pro Asp Gln Ile His Leu Ser Asn
                995                 1000                1005

aca aga gca cta caa tca cag ttt gca tcc tgg tat gaa ggt gtg aaa 3072
Thr Arg Ala Leu Gln Ser Gln Phe Ala Ser Trp Tyr Glu Gly Val Lys
            1010                1015                1020

aat gac tat gat gtc aca gat gaa cag atg caa ata ata ctg aat gga 3120
Asn Asp Tyr Asp Val Thr Asp Glu Gln Met Gln Ile Ile Leu Asn Gly
1025 1030 1035 1040

ttg atg gtt tgg tgt att gag aat ggg act tca cca aac att aat ggc 3168
Leu Met Val Trp Cys Ile Glu Asn Gly Thr Ser Pro Asn Ile Asn Gly
1045 1050 1055

tac tgg gtt atg atg gat gga gat gaa caa gtt gaa tac cca ata aaa 3216
Tyr Trp Val Met Met Asp Gly Asp Glu Gln Val Glu Tyr Pro Ile Lys
1060 1065 1070

cca tta atc gac cat gcc aaa cca tca ttt aga caa atc atg gca cac 3264
Pro Leu Ile Asp His Ala Lys Pro Ser Phe Arg Gln Ile Met Ala His
                1075                1080                1085

ttt agt aat ctt gct gaa gcg tac att gag aag cgc aat tct gag aag 3312
Phe Ser Asn Leu Ala Glu Ala Tyr Ile Glu Lys Arg Asn Ser Glu Lys
            1090                1095                1100

cct tat atg cca aga tat ggg ctt caa aga aac ctt acc gat atg tca 3360
Pro Tyr Met Pro Arg Tyr Gly Leu Gln Arg Asn Leu Thr Asp Met Ser
1105 1110 1115 1120

tta gcg cga tat gct ttc gat tta tac gaa atg aca tcg aaa act ccc 3408
Leu Ala Arg Tyr Ala Phe Asp Leu Tyr Glu Met Thr Ser Lys Thr Pro
1125 1130 1135

gtc cga gct cgt gag gca cac att caa atg aaa gca gct gcg tta cgt 3456
Val Arg Ala Arg Glu Ala His Ile Gln Met Lys Ala Ala Ala Leu Arg
1140 1145 1150

ggc gta agc aac agg atg ttt gga ctg gac ggt agg gta ggc aca cag 3504
Gly Val Ser Asn Arg Met Phe Gly Leu Asp Gly Arg Val Gly Thr Gln
                1155                1160                1165

gaa gag gac act gaa cgg cac aca gca gag gat gta aat aga aac atg 3552
Glu Glu Asp Thr Glu Arg His Thr Ala Glu Asp Val Asn Arg Asn Met
            1170                1175                1180

cat aat ctg ctg ggc gtt cga gga ttg tgaacttcaa cttttaacta 3599
His Asn Leu Leu Gly Val Arg Gly Leu
        1185                1190

gtaataagta acgtattgta gtatatgtaa cttggtttat gttgttgatc atacatattc 3659

ttcagtgtgg ctcccaccat aagttatgtg tgcttaactt catactactt tatatatatg 3719

ttatttaaaa ctttttactt aggtaactgt gtacccccgc ccattttcag cgtgttccca 3779

ccttaatgga atggtgcaac ccagactatt gttatggttt aaggagcgac cctttaggtc 3839

gttcaagcta ttcaatagtg gcg 3862

<210> 9
<211> 170
<212> PRT
<213> pepper vein banding virus

<400> 9
Ser Lys Gly Lys Thr His Gly Met Gly Lys Lys Asn Arg Arg Phe Val
1               5                   10                  15

Asn Met Tyr Gly Phe Asp Pro Thr Glu Tyr Ser Phe Ile Arg Phe Val
            20                  25                  30

Asp Pro Val Thr Gly Glu Met Leu Asp Glu Ser Val Met Ala Asp Val
        35                  40                  45

Met Leu Val Gln Glu His Phe Asn Asp Leu Arg Phe Glu Tyr Leu Gly
    50                  55                  60

Asp Asp Lys Ile Glu Ser Ala Arg Leu Tyr Ser Asn Pro Gly Ile Gln
65                  70                  75                  80

Ala Tyr Phe Val Lys Asp Lys Val Ser Pro Val Leu Lys Val Asp Leu
                85                  90                  95

Thr Gln His Val Pro Leu Lys Val Cys Asp Asn Ser Ser Thr Ile Ala
            100                 105                 110

Gly Phe Pro Glu Arg Glu Gly Glu Phe Arg Gln Thr Gly Gln Ala Thr
        115                 120                 125

Lys Val Asn Tyr Asp Glu Leu Pro Ser Arg Glu Gly Val Glu His Glu
    130                 135                 140

Ala Arg Ser Leu Asn Arg Gly Leu Arg Asp His Asn Gln Val Ser Lys
145                 150                 155                 160

Leu Ile Cys Lys Leu Glu Asn Asp Ser Ala
                165                 170

<210> 10
<211> 25
<212> PRT
<213> pepper vein banding virus

<400> 10
Leu Cys Tyr Leu Asn Ser Trp Cys Trp Leu Arg Val His Tyr Asn Tyr
1               5                   10                  15

Lys Ser Ser Phe Thr Gln Lys Glu Gln
            20                  25

<210> 11
<211> 996
<212> PRT

<213> pepper vein banding virus

<400> 11
Thr Leu Arg Val Lys Thr Ala His Gly Asp Phe Lys Val Ala Asn Thr
1               5                   10                  15

Lys Glu Met Lys Val Phe Pro Val Glu Lys His Asp Ile Leu Leu Ile
            20                  25                  30

Arg Leu Pro Lys Asp Phe Pro Pro Phe Pro Val Lys Ser Lys Phe Arg
        35                  40                  45

Glu Pro Lys Val Asn Asp Ser Ile Cys Leu Val Gly Thr Asn Phe Gln
    50                  55                  60

Glu Lys Phe Leu Ser Ser Leu Ile Ser Ala Asp Ser Thr Thr Ser Pro
65                  70                  75                  80

Val Ser Gly Ser Lys Phe Trp Arg His Trp Ile Asp Thr Lys Asp Gly
                85                  90                  95

His Cys Gly Leu Pro Leu Val Ala Arg Asp Asp Gly Ala Ile Val Gly
            100                 105                 110

Phe His Ser Leu Thr Ser Ile Asn Thr Glu Gln Asn Tyr Phe Ala Ala
        115                 120                 125

Val Pro Glu Ala Phe Met Glu Leu Ile Ala Gln Val Glu Thr Leu Glu
    130                 135                 140

Trp Arg Lys Ser Trp Val Tyr Asn Pro Asn Glu Ile Gly Trp Gly Ser
145                 150                 155                 160

Leu Lys Leu Lys Ser Asp Gln Pro Thr Gly Met Phe Lys Ile Glu Lys
                165                 170                 175

Leu Ile Glu Asp Ile Gln Ser Ala Phe Val Arg Glu Gln Ala Ser Asp
            180                 185                 190

Lys Trp Leu Tyr Ala Gln Phe Gln Gly Phe Leu Lys Ala Val Pro Lys
        195                 200                 205

Ser Glu Ser Gln Leu Val Pro Lys His Ile Val Lys Gly Pro Cys Pro
    210                 215                 220

Leu Phe His Leu Tyr Leu Ser Thr His Arg Glu Ala Arg Gln Phe Phe
225                 230                 235                 240

Gln Pro Phe Met Gly Ala Tyr Gly Lys Ser Arg Leu Asn Arg Ile Ala
                245                 250                 255

Tyr Ala Lys Asp Ile Leu Lys Tyr Ser Thr Glu Ile Glu Val Gly Lys
            260                 265                 270

Val Asp Thr Cys Ala Leu Glu Leu Pro Val Asp Asp Val Ile Ala Asp
        275                 280                 285

Leu His Ala Val Lys Phe Asp Thr Cys Asn Tyr Val Thr Asp Glu Glu
    290                 295                 300

Glu Ile Phe Gln Ser Leu Asn Met Lys Ala Ala Val Gly Ala Leu Tyr
305                 310                 315                 320

Lys Gly Lys Lys Arg Glu Tyr Phe Glu Thr Tyr Thr Gln Gln Asp Lys
                325                 330                 335

Glu Arg Ile Leu Phe Glu Ser Cys Leu Arg Leu Tyr Lys Gly Asn Leu
            340                 345                 350

Gly Val Trp Asn Gly Ser Ile Lys Ala Glu Leu Arg Pro Ile Glu Lys
        355                 360                 365

Val Arg Ala Asn Lys Thr Arg Thr Phe Thr Ala Ala Pro Leu Asp Thr
    370                 375                 380

Leu Leu Ala Gly Lys Val Cys Val Asp Asp Phe Asn Asn Gln Phe Tyr
385                 390                 395                 400

Ser Lys His Thr Leu Ala Pro Trp Ser Val Gly Ile Ser Lys Phe Ser
                405                 410                 415

Gly Gly Trp Asp Arg Leu Leu Arg Lys Leu Pro Asp Gly Trp Ile Tyr
            420                 425                 430

Cys Ser Ala Asp Gly Ser Arg Phe Asp Ser Ser Leu Thr Pro Tyr Leu
        435                 440                 445

Ile Asn Ala Val Ala Ser Ile Arg Leu Lys Phe Met Glu Pro Trp Gly
    450                 455                 460

Ile Gly Glu Gln Met Leu Met Arg Asn Leu Tyr Ala Glu Ile Ile Tyr
465                 470                 475                 480

Thr Pro Ile Leu Thr Ala Asp Gly Thr Ile Val Lys Lys Ser Lys Gly
                485                 490                 495

Asn Asn Ser Gly Gln Pro Ser Thr Val Val Asp Asn Thr Leu Met Val
            500                 505                 510

Leu Val Ala Met Lys Tyr Ser Leu Arg Arg Leu Gly Ile Ile Ile Arg
        515                 520                 525

Ile Lys Asn Asp Arg Gly Val Phe Phe Ala Asn Gly Asp Asp Leu Ile
    530                 535                 540

Val Ser Val Pro Pro Ser Asp Glu Trp Ile Leu Asp Ser Leu Gln Asp
545                 550                 555                 560

Arg Ser Ser Glu Leu Gly Leu Ser Tyr Asp Phe Asn Glu Arg Thr Thr
                565                 570                 575

Asp Arg Ser Glu Leu Trp Phe Met Ser His Gln Gly Ile Leu Ile Glu
            580                 585                 590

Asn Gln Tyr Ile Pro Lys Leu Glu Pro Glu Arg Ile Val Ser Ile Leu
        595                 600                 605

Glu Trp Asp Arg Ala Glu Gln Pro Glu His Arg Leu Glu Ala Ile Cys
    610                 615                 620

Ala Ser Met Ile Glu Ala Trp Gly His Lys Glu Leu Leu Tyr Glu Ile
625                 630                 635                 640

Arg Leu Phe Tyr Lys Trp Val Ile Glu Gln Ala Pro Tyr Ser Gln Ile
                645                 650                 655

Val Ser Glu Gly Lys Ala Pro Tyr Ile Ser Glu Thr Ala Leu Arg Cys
            660                 665                 670

Leu Asn Met Ser Glu His Gly Glu Asn Asp Ile Asn Pro Tyr Leu Arg
        675                 680                 685

Ala Leu Ile Glu Gly Ala Lys Arg Glu Glu Leu Asp Asp Asp Gly Gly
    690                 695                 700

Glu Val Ala His Gln Ala Gly Glu Ser Val Asp Ala Gly Ser Val Lys
705                 710                 715                 720

Gly Glu Asp Ser Ser Ser Lys Ser Ala Asp Lys Gln Ala Thr Glu Lys
                725                 730                 735

Lys Asn Lys Val Ser Gly Gln Ala Gln Pro Gln Ser Arg Gln Ser Glu
            740                 745                 750

Met Glu Val Pro Gln Val Arg Asp Arg Asp Val Asn Val Gly Thr Ser
        755                 760                 765

Gly Thr Phe Thr Ile Pro Arg Leu Lys Gly Ile Phe Ser Lys Leu Thr
    770                 775                 780

Ile Pro Lys Val Lys Thr Lys Ala Val Val Asn Leu Glu His Leu Leu
785                 790                 795                 800

Asp Tyr Ala Pro Asp Gln Ile His Leu Ser Asn Thr Arg Ala Leu Gln
                805                 810                 815

Ser Gln Phe Ala Ser Trp Tyr Glu Gly Val Lys Asn Asp Tyr Asp Val
            820                 825                 830

Thr Asp Glu Gln Met Gln Ile Ile Leu Asn Gly Leu Met Val Trp Cys
        835                 840                 845

Ile Glu Asn Gly Thr Ser Pro Asn Ile Asn Gly Tyr Trp Val Met Met
    850                 855                 860

Asp Gly Asp Glu Gln Val Glu Tyr Pro Ile Lys Pro Leu Ile Asp His
865                 870                 875                 880

Ala Lys Pro Ser Phe Arg Gln Ile Met Ala His Phe Ser Asn Leu Ala
                885                 890                 895

Glu Ala Tyr Ile Glu Lys Arg Asn Ser Glu Lys Pro Tyr Met Pro Arg
            900                 905                 910

Tyr Gly Leu Gln Arg Asn Leu Thr Asp Met Ser Leu Ala Arg Tyr Ala
        915                 920                 925

Phe Asp Leu Tyr Glu Met Thr Ser Lys Thr Pro Val Arg Ala Arg Glu
    930                 935                 940

Ala His Ile Gln Met Lys Ala Ala Ala Leu Arg Gly Val Ser Asn Arg
945                 950                 955                 960

Met Phe Gly Leu Asp Gly Arg Val Gly Thr Gln Glu Glu Asp Thr Glu
                965                 970                 975

Arg His Thr Ala Glu Asp Val Asn Arg Asn Met His Asn Leu Leu Gly
            980                 985                 990

Val Arg Gly Leu
        995

<210> 12
<211> 864
<212> DNA
<213> pepper vein banding virus

<400> 12
gcaggagaga gtgttgatgc tgggagcgta aaaggcgaag actcatcaag caaatcagct 60
gataaacaag ccacagagaa aaagaacaaa gtgagtggac aagctcagcc acagtctcgt 120
cagagtgaaa tggaagtacc ccaagttcga taagggtatt ttttcaaagt taacaatacc 180
aaaggtcaaa acaaaagctg ttgttaacct tgaacacctt cttgattatg ctcctgatca 240
aatacatttg ttgttaacct tgaacacctt cttgattatg ctcctgatca aatacatttg 300
agtaacacaa gagcactaca atcacagttt gcatcctggt atgaaggtgt gaaaaatgac 360
tatgatgtca cagatgaaca gatcgaaata atactgaatg gattgatggt ttggtgtatt 420
gagaatggga cttcaccaaa cattaatggc tactgggtta tgatggatgg agatgaacaa 480
gttgaatacc caataaaacc attaatcgac catgccaaac catcatttag acaaatcatg 540
gcacacttta gtaatcttgc tgaagcgtac attgagaagc gcaattctga gaagccttat 600
atgccaagat atgggcttca aagaaacctt accgatatgt cattagcgcg atatgctttc 660
gatttatacg aaatgacatc gaaaactccc gtccgagctc gtgaggcaca cattcaaatg 720
aaagcagctg cgttacgtgg cgtaagcaac aggatgtttg gactggacgg tagggtaggc 780
acacaggaag aggacactga acggcacaca gcagaggatg taaatagaaa catgcataat 840
ctgctgggcg ttcgaggatt gtga 864

<210> 13
<211> 287
<212> PRT
<213> pepper vein banding virus

<400> 13
Ala Gly Glu Ser Val Asp Ala Gly Ser Val Lys Gly Glu Asp Ser Ser
1               5                   10                  15

Ser Lys Ser Ala Asp Lys Gln Ala Thr Glu Lys Lys Asn Lys Val Ser
            20                  25                  30

Gly Gln Ala Gln Pro Gln Ser Arg Gln Ser Glu Met Glu Val Pro Gln
        35                  40                  45

Val Arg Asp Arg Asp Val Asn Val Gly Thr Ser Gly Thr Phe Thr Ile
    50                  55                  60

Ala Arg Leu Lys Gly Ile Phe Ser Lys Leu Thr Ile Pro Lys Val Lys
65                  70                  75                  80

Thr Lys Ala Val Val Asn Leu Glu His Leu Leu Asp Tyr Ala Pro Asp
                85                  90                  95

Gln Ile His Leu Ser Asn Thr Arg Ala Leu Gln Ser Gln Phe Ala Ser
            100                 105                 110

Trp Tyr Glu Gly Val Lys Asn Asp Tyr Asp Val Thr Asp Glu Gln Met
        115                 120                 125

Gln Ile Ile Leu Asn Gly Leu Met Val Trp Cys Ile Glu Asn Gly Thr
    130                 135                 140

Ser Pro Asn Ile Asn Gly Tyr Trp Val Met Met Asp Gly Asp Glu Gln
145                 150                 155                 160

Val Glu Tyr Pro Ile Lys Pro Leu Ile Asp His Ala Lys Pro Ser Phe
                165                 170                 175

Arg Gln Ile Met Ala His Phe Ser Asn Leu Ala Glu Ala Tyr Ile Glu
            180                 185                 190

Lys Arg Asn Ser Glu Lys Pro Tyr Met Pro Arg Tyr Gly Leu Gln Arg
        195                 200                 205

Asn Leu Thr Asp Met Ser Leu Ala Arg Tyr Ala Phe Asp Leu Tyr Glu
    210                 215                 220

Met Thr Ser Lys Thr Pro Val Arg Ala Arg Glu Ala His Ile Gln Met
225                 230                 235                 240

Lys Ala Ala Ala Leu Arg Gly Val Ser Asn Arg Met Phe Gly Leu Asp
                245                 250                 255

Gly Arg Val Gly Thr Gln Glu Glu Asp Thr Glu Arg His Thr Ala Glu
            260                 265                 270

Asp Val Asn Arg Asn Met His Asn Leu Leu Gly Val Arg Gly Leu
        275                 280                 285